# EUROPEAN PATENT APPLICATION

(11) **EP 2 082 710 A2**
(43) Date of publication of application: **29.07.2009**
(21) Application number: 08170462.9
(22) Date of filing: 02.12.2008
(51) Int. Cl.: A61F 7/03

(54) **Kit comprising exothermic structure and at least two pressure-sensitive adhesive structures**

(30) Priority: 25.01.2008 US 19998
(71) Applicant: Ohshin MLP Co., Ltd, Echizen-shi Fukui 915-0052 (JP)
(72) Inventor: Ota, Keizo, Fukui Fukui 915-0052 (JP); Suzuki, Noriko, Tokyo Tokyo 104-0051 (JP)
(74) Representative: Giovannini, Francesca

(57) **Abstract**

A kit that includes an exothermic structure, at least two pressure-sensitive adhesive structures, and an air-impermeable bag is used to make an exothermic structure having at least two pressure-sensitive adhesive structures. The exothermic structure includes a pouch having an air-permeable side and an air-impermeable side, and an exothermic composition. The adhesive structures each comprise a base, an adhesive layer, and a cover in this order. To make the exothermic structure, parts of the covers are peeled off to expose parts of surfaces of the adhesive layers, and then the exposed adhesive layers are adhered to ends of the air-permeable side of the exothermic structure.

## Description

### BACKGROUND OF INVENTION

### Field of the Invention

The present invention relates to an exothermic kit and use thereof, wherein the kit comprising an exothermic structure, at least two pressure-sensitive adhesive structures, and an air-impermeable bag, wherein before use the adhesive structures are adhered to ends of the exothermic structure.

### Background Art

Disposable exothermic structures or devices, i.e., disposable body warmers, are convenient devices for warming up a part of human body. Among the exothermic structures, some are used by directly applying to skin with a pressure-sensitive adhesive.

Usually, the adhesive is applied all over one surface of an exothermic structure. When such an exothermic structure is peeled from the skin after use, body hairs are pulled, leading to a tingle sensation, discomfort, or rough skin areas. Some users may also suffer from low-temperature bums because the entire exothermic structure is tightly adhere to the skin.

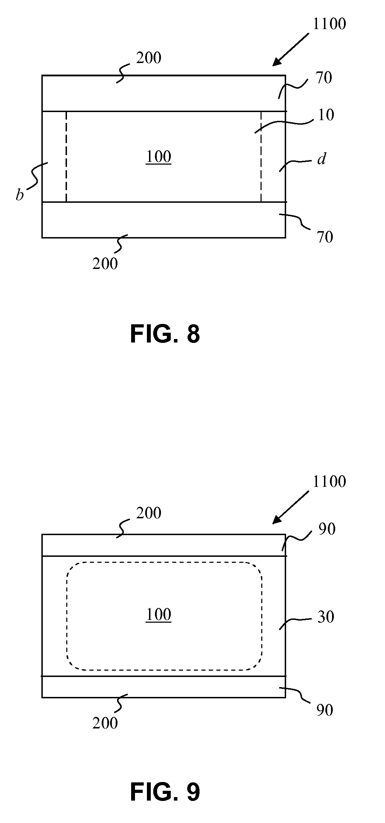

Japanese Patent Early-publication No. 2006-271963 (hereafter "the '963 application") discloses a device for improving physiological functions. Such a device comprises an exothermic part that uses chemical energy to supply water vapor generated from the exothermic part to skin. Fig. 8 of the '963 application illustrates one such device. In Fig. 8, the adhesive layers 7, 7, i.e., parts for sticking, are applied at ends 6b, 6b.

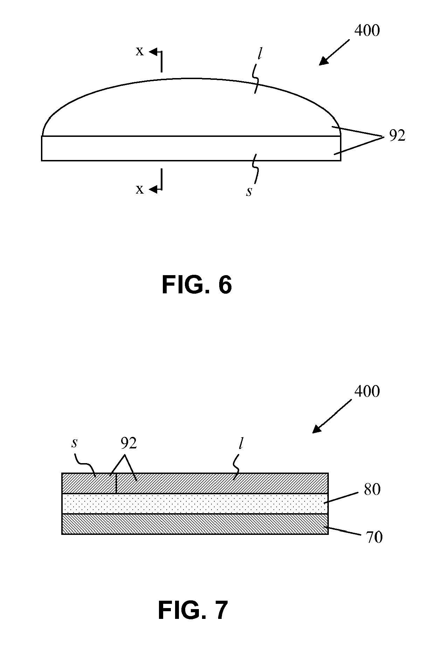

Japanese Patent Early-publication No. 2007-90057 (hereafter "the '057 application") discloses a device for supplying water vapor using heat generated from an exothermic part. Fig. 6 of the '057 application discloses one such device. In Fig. 6, the adhesive layers N, N are also applied at the ends.

In the above devices, fewer body hairs are pulled when the device is removed after use, producing only tingle sensation to the user (without significant discomfort). Furthermore, the risk of low-temperature bum is lower with these devices.

In the above devices, chemical energies are generated by reacting iron powder with oxygen. In the preparation of these devices, after iron powder has been put in an air-permeable pouch and the pouch has been sealed, the pouch should be promptly put into an outer bag that is air-impermeable, and the outer bag should be promptly sealed to prevent reactions between iron powder and oxygen. Therefore, it is difficult to prepare the adhesive layers on the pouch after the iron powder has been put in the pouch.

An alternative approach is to use a sheet material for pouches that includes adhesive layers at both longitudinal ends. However, in this case, it is difficult to heat-seal the sheet material that will become one side of the pouch with another sheet material that will become the other side of the pouch. Thus, it is difficult to prepare the above exothermic devices, and to overcome those difficulties, expensive machines are often needed.

### SUMMARY OF INVENTION

The present invention intends to provide kits that each comprises parts by which an exothermic structure having at least two pressure-sensitive adhesive parts on its ends can be easily made by users. The exothermic structure can be directly applied to skin to warm up a part of human body. The exothermic structure produces less tingle or discomfort when peeled off after use and have a lower risk of low-temperature bum. The kits can be prepared at low costs. A kit in accordance with one embodiment of the invention comprises an exothermic structure, at least two pressure-sensitive adhesive structures, and an air-impermeable bag. Further, the present invention intends to provide methods for using the above kit.

The present inventors have studied diligently to attain the above objectives. As a result, they have accomplished the present invention.

Namely, the present invention provides kits each comprising an exothermic structure, at least two pressure-sensitive adhesive structures, and an air-impermeable bag, wherein the exothermic structure comprises a pouch having an air-permeable side and an air-impermeable side, and an exothermic composition that is sealed in the pouch, wherein the pressure-sensitive adhesive structures each comprise a base, a pressure-sensitive adhesive layer, and a cover in this order, and wherein the exothermic structure, with or without the adhesive structures, is sealed in the air-impermeable bag.

Further, the present invention provides methods for using the above kits. A method in accordance with one embodiment of the invention includes the steps of taking the exothermic structure out of the air-impermeable bag, peeling off parts of the covers of the pressure-sensitive adhesive structures to expose parts of surfaces of the pressure-sensitive adhesive layers, pressing the bases of the adhesive structures to adhere the exposed adhesive layers to ends of the air-permeable side of the exothermic structure, peeling off the remaining covers to expose the remaining surfaces of the adhesive layers, and pressing the bases of the adhesive structures to adhere the exposed adhesive layers to a site of application, such as skin or outside of underwear.

Other aspects and advantages of the invention will be apparent from the following description and the appended claims.

### BRIEF DESCRIPTION OF DRAWINGS

Figure 1 is plane views of examples of parts that are contained in a kit according to one embodiment of the present invention.

Figure 2 is a sectional view of one example of an exothermic structure as a part of a kit according to one embodiment of the present invention.

Figure 3 is a sectional view of one example of a pressure-sensitive adhesive structure as a part of a kit according to one embodiment of the present invention.

Figure 4 is a plane view of another example of an exothermic structure as a part of a kit according to one embodiment of the present invention.

Figure 5 is a plane view of another example of an exothermic structure as a part of a kit according to one embodiment of the present invention.

Figure 6 is a plane view of another example of a pressure-sensitive adhesive structure as a part of a kit according to one embodiment of the present invention.

Figure 7 is a sectional view of the adhesive structure of Figure 6 at the X-X line.

Figure 8 is a plane view of an exothermic structure having two pressure-sensitive adhesive parts. The structure was made by using an exothermic structure and pressure-sensitive adhesive structures shown in Figure 1.

Figure 9 is a back view of the exothermic structure having two pressure-sensitive adhesive parts shown in Figure 8.

Figure 10 is a plane view of another example of an exothermic structure having two pressure-sensitive adhesive parts. The structure was made by using an exothermic structure and pressure-sensitive adhesive structures shown in Figure 1.

Figure 11 is a plane view of an exothermic structure having three pressure-sensitive adhesive parts. The structure was made by using an exothermic structure and the pressure-sensitive adhesive structures shown in Figure 1.

### DETAILED DESCRIPTION

Hereafter, the present invention will be explained with reference to preferred examples. Note that these examples are for illustration only and are not intended to limit the scope of the invention.

First, with reference to the figures, the physical components of a kit according to embodiments of the present invention will be explained.

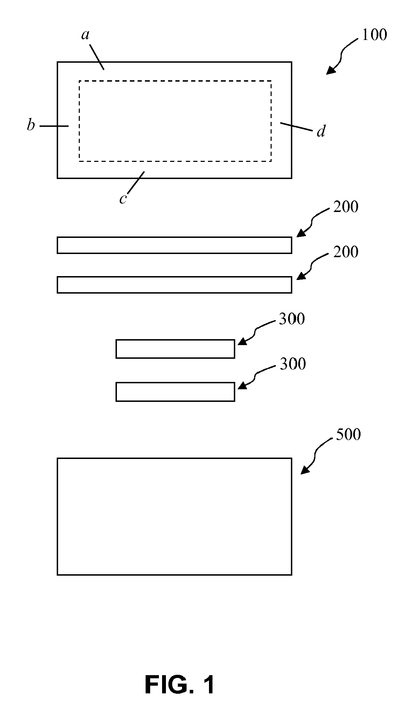

Figure 1 shows a set of parts (or components) contained in a kit according to one embodiment of the present invention. In this example, the kit comprises an exothermic structure 100, pressure-sensitive adhesive structures 200, 200, 300, 300, and an air-impermeable bag 500. The exothermic structure 100 is put in the sealed air-impermeable bag 500 until use. The adhesive structures 200, 200, 300, 300 may also be put in the air-impermeable bag 500. Alternatively, the adhesive structures 200, 200, 300, 300 may be placed outside of the air-impermeable bag 500. If the adhesive structures 200, 200, 300, 300 are not put in the air-impermeable bag 500, all the parts of the kit may be put into another container, e.g., a box or a bag.

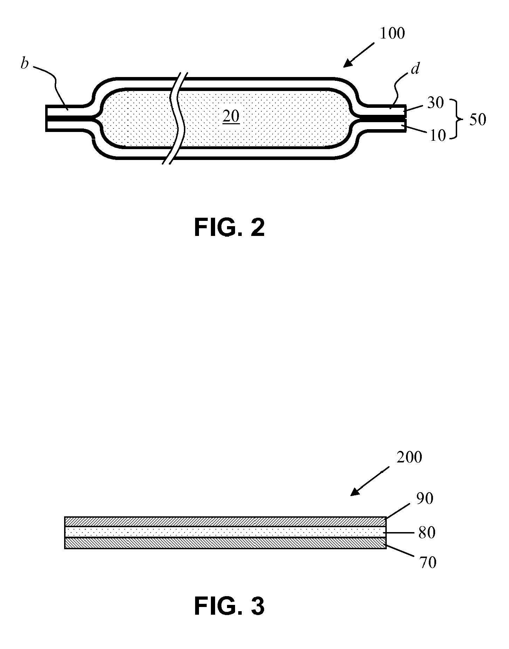

As shown in Figure 2, the exothermic structure 100 may comprise a pouch 50 having an air-permeable side 10 and an air-impermeable side 30, and an exothermic composition 20 that is placed inside the sealed pouch 50.

The exothermic structure 100 has four ends (or edges or border portions) a, b, c, and d that are shown as areas outside of the dotted lines in Figure 1. The ends a, b, c, and d are border portions of the pouch 50 and do not comprise the chamber that holds the exothermic composition.

In this example, the adhesive structures 200, 200, 300, 300 each comprise a base 70, a pressure-sensitive adhesive layer 80, and a cover 90 in this order, as shown in Figure 3.

The air-impermeable bag 500 has a size and shape that is configured to accommodate the exothermic structure 100.

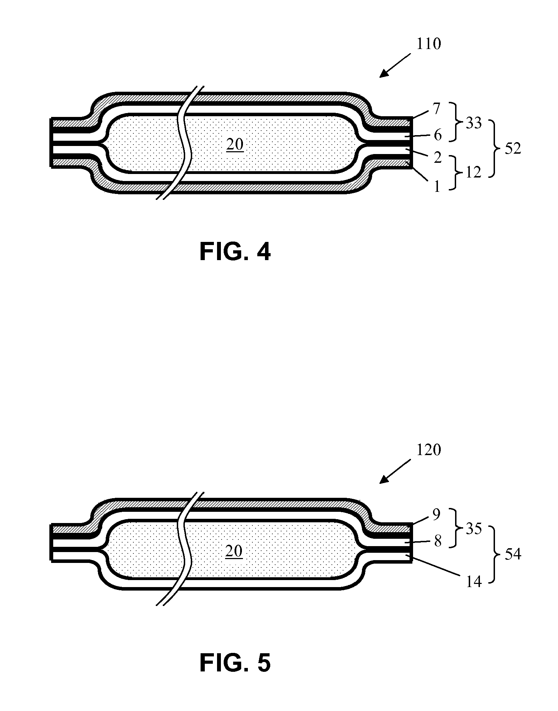

In an exothermic structure according to embodiments of the present invention, the air-permeable side of the pouch may be made of single-layered materials, as shown in Figure 2. Alternatively, it may be constructed of two or more layered materials. For example, the air-permeable side 12 of the pouch 52 in the exothermic structure 110 shown in Figure 4 is constructed of layers 1 and 2. The layer 1 may be a fiber layer that is made of, e.g., non-woven fabric, woven fabric, or knitted fabric. The layer 2 may be a porous polymer layer. The layer 1 may be partly bonded to the layer 2 to maintain air permeability.

In an exothermic structure according to embodiments of the present invention, the air-impermeable side of the pouch may be made of single-layered materials, as shown in Figure 2. Alternatively, it may be constructed of two or more layered materials. For example, the air-impermeable side 33 of the pouch 52 in the exothermic structure 110 shown in Figure 4 is constructed of layers 6 and 7. Furthermore, the air-impermeable side 35 of the pouch 54 in an exothermic structure 120 shown in Figure 5 is constructed of layers 8 and 9. The layers 6 and 8 each may be an air-impermeable polymer layer. The layers 7 and 9 each may be a fiber layer that is made of, e.g., non-woven fabric, woven fabric, or knitted fabric. Alternatively, the layers 7 and 9 each may also be an air-impermeable polymer layer. The layer 6 may be entirely or partly bonded to the layer 7. The layer 8 may be entirely or partly bonded to the layer 9.

In accordance with embodiments of the invention, it is preferable that the air-permeable side can be visually distinguished from the air-impermeable side. If their appearances are different, users can readily identify the side, to which the pressure-sensitive adhesive structures should be adhered, i.e., the air-permeable side.

For example, the pouch 54 in the exothermic structure 120 of Figure 5 has a porous polymer layer as the air-permeable side 14 and a fabric layer (e.g., a non-woven fabric layer) as an outer layer 9 of the air-impermeable side 35. Because of the different materials, users can distinguish the air-permeable side from the air-impermeable side.

It is also preferable that an outer surface of the air-permeable side and that of the air-impermeable side have different colors and/or patterns. Particularly preferably, an outer surface of the base of the pressure-sensitive adhesive structure has a color and/or pattern that is the same as the outer surface of the air-permeable side of the pouch in the exothermic structure. With these embodiments, the users can properly adhere the adhesive structure to the air-permeable side of the exothermic structure.

For a pressure-sensitive adhesive structure, the cover may be one piece, as shown in Figure 1. However, as shown in Figures 6 and 7, the cover 92 of the pressure-sensitive adhesive structure 400 is preferably cut to give a small piece *s* and a large piece *l*. This is because only the part of the surface of the pressure-sensitive adhesive layer that is used to adhere the adhesive structure to an exothermic structure can be readily exposed. In the pressure-sensitive adhesive structure 400 shown in Figure 7, a part of the pressure-sensitive adhesive layer 80 (i.e., the part facing the small piece *s* of the cover 92) may be used to adhere the adhesive structure 400 to an exothermic structure, and the remaining part that faces the large piece *l* of the cover 92 may be used to adhere the whole exothermic structure to skin or outside of underwear, for example. Note that the description of small piece *s* and large piece *l* is for clarity of illustration. One of ordinary skill in the art would appreciate that these parts may be of equal size, or may have more than two parts, without departing from the scope of the invention.

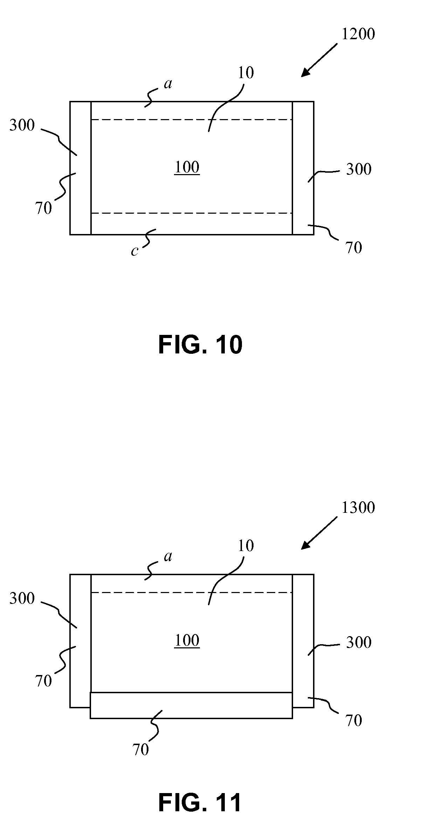

A kit according to embodiments of the present invention comprises at least two pressure-sensitive adhesive structures. For example, as shown in Figures 8 and 9, two pressure-sensitive adhesive structures 200, 200 may be adhered to ends *a* and *c* of the air-permeable side 10 of the exothermic structure 100. As shown in Figure 10, two pressure-sensitive adhesive structures 300, 300 may be adhered to ends *b* and *d* of the air-permeable side 10 of the exothermic structure 100. As shown in Figure 11, two pressure-sensitive adhesive structures 300, 300 may be adhered to ends *b* and *d* of the air-permeable side 10 of the exothermic structure 100, and another pressure-sensitive adhesive structure that was shortened by cutting a portion of the adhesive structure 200 may be adhered to end *c* of the air-permeable side 10 of the exothermic structure 100. Of course, four pressure-sensitive adhesive structures may be adhered to ends *a, b, c,* and *d* of the air-permeable side 10 of the exothermic structure 100. Alternatively, two or more pressure-sensitive adhesive structures may be adhered to one end.

The embodiments shown in Figures 8 through 11 are examples. One of ordinary skill in the art would appreciate that other modifications are possible without departing from the scope of the invention.

Next, a method for using a kit of the present invention will be explained.

A method in accordance with one embodiment of the invention may comprise: (1) taking the exothermic structure out of an air-impermeable bag, (2) peeling off parts of the covers of the pressure-sensitive adhesive structures to expose parts of the surfaces of the pressure-sensitive adhesive layers, (3) pressing the bases of the adhesive structures to adhere the exposed adhesive layers to ends of the air-permeable side of an exothermic structure, (4) peeling off the remaining covers to expose the remaining surfaces of the adhesive layers, and (5) pressing the bases of the adhesive structures to adhere the exposed adhesive layers to a site of application (e.g., skin or outside of underwear).

First, a user opens an air-impermeable bag and then takes out the exothermic structure from the air-impermeable bag (step 1). After step 1, air may pass through the air-permeable side of the pouch of the exothermic structure and an exothermic reaction may start in the exothermic composition.

Then, the user peels off a part of the cover of the pressure-sensitive adhesive structure to expose a part of the surface of the pressure-sensitive adhesive layer (step 2). At this point, the part of the cover that was peeled off may be folded back (if the cover is not cut into separate pieces as shown in Figures 6 and 7) and the rest of the cover remains on the adhesive layer. If a cover having a small piece *s* and large piece *l* is used, the small piece *s* (or the large piece *l*) may be peeled off and removed first.

By pressing the base of the adhesive structure, the exposed adhesive layer may be adhered to an end of the air-permeable side of the exothermic structure (step 3).

The user performs steps 2 and 3 for individual adhesive structures that are used. By performing steps 1 through 3, an exothermic structure having two or more pressure-sensitive adhesive structures can be obtained. Such examples are shown as structures 1,100, 1,200, and 1,300 in Figures 8 through 11.

The user peels off the rests of the covers to expose the remaining surfaces of the adhesive layers (step 4). Then, the user presses the bases of the adhesive structures to adhere the exposed adhesive layers to a site of application (e.g., skin or outside of underwear) (step 5). The user may perform steps 4 and 5 at the same time. Namely, the user may adhere the adhesive layer to skin or outside of underwear while peeling off the rest of the cover. Alternatively, the user may peel off a first cover and adhere a first adhesive structure to the application site (e.g., skin) before peeling off a second cover from a second adhesive structure and adhering the second adhesive structure to the application site, and so on. Again, the description of the order of the steps are for illustration only, and one of ordinary skill in the art would appreciate that other modifications and variations are possible without departing from the scope of the invention.

Next, materials and the like that constitute the parts of a kit according to embodiments of the present invention will be explained.

The exothermic composition of an exothermic structure may generate heat by reaction with air (e.g., oxygen). The components that are contained in an exothermic composition are not particularly limited, as long as they are conventionally used in exothermic compositions that can generate heat by reaction with air. Examples of such components are as follows.

Examples of chemical exothermic agents include metal powders such as iron powders (reduced iron powder, atomized iron powder, and the like). The exothermic compositions may also include reaction auxiliaries. Examples of reaction auxiliaries include metal halides such as sodium chloride, potassium chloride, magnesium chloride, calcium chloride, iron (II) chloride, and iron (III) chloride; and metal sulfates such as potassium sulfate, sodium sulfate, magnesium sulfate, copper sulfate, iron (II) sulfate, and iron (III) sulfate. The exothermic compositions may also include water retaining agents. Examples of water retaining agents include active carbon, alumina, silica gel, zeolite, wood charcoal, and water-absorptive polymeric compounds. Of course, water may also be used. The exothermic compositions may also include other additives. Examples of other additives include polymeric compounds such as carboxymethyl cellulose, acrylic acid starch, polyethylene, polypropylene, and polystyrene; bentonite; vermiculite; pearlite; and wood charcoal.

In accordance with one embodiment of the invention, it is preferable that an exothermic composition that generates heat by reaction with air is formulated in a manner such that a metal powder (such as an iron powder) has a less tendency to aggregate or shift around in the pouch. For example, such formulation may comprise a polymeric compound (e.g., carboxymethyl cellulose) and water. Using a polymeric compound that has been wet with water, the metal powder can be held in place. Therefore, the metal powder would not tend to aggregate or shift around in the pouch.

An exothermic composition in accordance with embodiments of the invention may be processed so as to be in a sheet-like form. In this case, the thickness may be within a range of preferably 5 mm or less, still more preferably from 0.5 to 4 mm, and particularly preferably from 1 to 2 mm.

An exothermic composition in accordance with embodiments of the invention may be put in a pouch having an air-permeable side and an air-impermeable side, and the pouch is sealed.

An example of the materials for the air-permeable side and the air-impermeable side includes a polymer film. Examples of raw materials that may be used to prepare the polymer film include polyolefin such as polyethylene and polypropylene; polyamides such as nylons; polyesters such as polyethylene terephthalate; ethylene copolymers such as ethylene-vinyl acetate copolymers and their saponified ones, and ethylene-alkyl (meth)acrylate copolymers; poly(vinyl chloride); poly(vinylidene chloride); polyurethanes; polystyrene; and polycarbonates. Furthermore, natural rubbers, reclaimed rubbers, and synthetic rubbers may also be used.

An example of the materials for an air-permeable side includes a moisture-permeable porous polymer film. In this description, the term "moisture-permeability" may be used to indicate air-permeability. If moisture can pass through a film, a gas (i.e., air) can also pass through the film. That is, a material having a moisture-permeability also has an air-permeability.

The porous polymer film can be prepared by making, to an air-impermeable polymer film, openings (holes) through which air may pass. To make the porous polymer film, for example, a polymer composition comprising inorganic particles may be used. After a polymer film comprising inorganic particles is prepared, stretching the film will produce openings in the film.

If an air-permeable side and an air-impermeable side are constructed of polymer films as described above, their thicknesses are preferably 100 µm or less, more preferably from 20 to 80 µm, and still more preferably from 40 to 60 µm.

If an air-permeable side or an air-impermeable side is constructed of two or more layered materials, a fiber layer that is made of, e.g., non-woven fabric, woven fabric, or knitted fabric may be used with a polymer film described above. Examples of raw materials for the fiber layer may include rayons, nylons, polyesters, acrylics, polypropylene, vinylon, polyethylene, polyurethanes, cotton, and celluloses.

The fiber layer is preferably made of a non-woven fabric. The non-woven fabric is preferably a spun lace or spun bond one. The non-woven fabric may have a basis weight of from 20 to 80 g/m², preferably from 30 to 70 g/m², and particularly preferably from 40 to 50 g/m².

If the fiber layer is made of a woven fabric or knitted fabric, it may have a basis weight of 200 g/m² or less, preferably from 20 to 120 g/m², and still more preferably from 40 to 100 g/m².

The two or more layered materials on the air-impermeable side may be constructed of different types of polymer layers.

It is preferable that at least one of the air-permeable side and the air-impermeable side is a polymer film having heat-sealability. If the air-permeable side or the air-impermeable side is constructed of two or more layers, at least the innermost layer is preferably a polymer film having heat-sealability. Examples of heat-sealable film include metallocene polyethylene film. Heat-sealability facilitates the formation of a sealed pouch.

Air-permeability of the air-permeable side affects the exothermic property, e.g., rates of heat generation. Air permeability may be represented by moisture-permeability [JIS Z 0208 (1976)] determined by the Lyssy method. In accordance with embodiments of the invention, it is preferable that the air-permeable side has a moisture-permeability of 200 to 500 g/m². 24 hours (preferably 250 to 400 g/m². 24 hours).

Examples of pressure-sensitive adhesive compositions that may constitute a pressure-sensitive adhesive layer of a pressure-sensitive adhesive structure include rubber-type adhesive compositions, acrylic-type adhesive compositions, and other adhesive compositions, each of which may comprise a thermoplastic resin (for example, a polyamide-type resin, a polyethylene-type resin, or a cellulose-type resin) as the main component.

Examples of pressure-sensitive adhesives that may be used in rubber-type adhesive compositions include diene-type polymeric compounds, for example natural rubber, synthetic rubbers, or mixtures thereof. Examples of synthetic rubbers include styrene-isoprene block copolymer rubber, styrene-isoprene-styrene block copolymer rubber, styrene-isobutylene-styrene block copolymer rubber, styrene-butadiene rubber, polyisoprene rubber, butyl rubber, chloroprene rubber, nitrile rubber, polysulfide rubber, and silicone rubber.

Examples of pressure-sensitive adhesives that may be used in the acrylic-type adhesive compositions include conventionally-used copolymers that contains at least one (meth)acrylate, such as n-butyl (meth)acrylate, hexyl (meth)acrylate, decyl (meth)acrylate, dodecyl (meth)acrylate, and tridecyl (meth)acrylate, and a functional or vinyl monomer that is copolymerable with the (meth)acrylate, for example, (meth)acrylic acid, maleic acid, maleic anhydride, hydroxyethyl acrylate, hydroxypropyl acrylate, acrylamide, dimethylacrylamide, aminoethyl methacrylate, methoxyethyl (meth)acrylate, acrylonitrile, vinyl acetate, and vinyl propionate.

The adhesive layer is prepared on a base. Thus, the form of an adhesive composition is not particularly limited, as long as it can be readily applied onto the base, for example, an emulsion, a solution with a solvent, an aqueous solution, a hot-melt type one, etc.

A base is preferably a fiber layer that is made of, e.g., a non-woven fabric, woven fabric, or knitted fabric. Examples of raw materials for the fiber layer may include rayons, nylons, polyesters, acrylics, polypropylene, vinylon, polyethylene, polyurethanes, cotton, and celluloses.

A fiber layer as the base is preferably made of a non-woven fabric. The non-woven fabric is preferably a spun lace or spun bond one. The non-woven fabric has a basis weight of usually from 20 to 80 g/m², preferably from 30 to 70 g/m², and particularly preferably from 40 to 50 g/m².

If the fiber layer as the base is made of a woven fabric or knitted fabric, it has a basis weight of usually 200 g/m² or less, preferably from 20 to 120 g/m², and still more preferably from 40 to 100 g/m².

A cover, in other word, a releasable sheet, in an adhesive structure covers the surface of a pressure-sensitive adhesive layer before use. The materials for a cover are not limited, as long as they are conventionally used in the field. For example, various plastic films, metal foils, and a laminate of a plastic film and paper may be used. A coating agent that facilitates the release of a cover from the adhesive layer, such as a silicone type, an alkylacrylate type, or a fluorine type coating agent, may be applied onto the inner surface of the cover that faces the adhesive layer. Examples of polymeric compounds that constitute the plastic films for the cover may include polyesters, polypropylene, polyethylene, alkylbenzene sulfonates, and poly(vinyl chloride).

An air-impermeable bag, into which the exothermic structure is placed, is typically constructed of a moisture resistant, air-impermeable material. Because the bag is air-impermeable, the exothermic agent in an exothermic composition does not chemically react, and thus the exothermic composition may be kept without generating heat. Examples of materials for the air-impermeable bag may include a laminate film of a cast polypropyrene with a polyethylene or a laminate of aluminum foil with a polymer film.

Next, one example of a process for preparing a kit in accordance with embodiments of the invention will be explained.

First, an air-permeable sheet (that will become the air-permeable side) is heat-sealed with an air-impermeable sheet (that will become the air-impermeable side) in a lateral direction and then in a longitudinal direction at both sides so as to make a pouch having three sealed sides (e.g., ends *b, c,* and *d* of the exothermic structure 100 in Figure 1) and one open side. Then, an exothermic composition is put into the pouch. Afterwards, heat-sealing in a lateral direction is performed to seal the pouch. The exothermic composition may be pressed into a layered form. At the center of the longitudinal direction of the laterally heat-sealed portion, cutting may be performed to separate individual exothermic structures. An exothermic structure and two or more pressure-sensitive adhesive structures that have been separately made may be put into an air-impermeable bag. Then, the bag is promptly sealed, e.g., by heat-sealing. As noted above, in accordance with some embodiments of the invention, an exothermic structure alone (i.e., without the adhesive structures) may be sealed in an air-impermeable bag by itself. This bag and the adhesive structures may be placed in a second bag, box, pouch, or the like, to form a kit of the invention.

The pressure-sensitive adhesive structure in accordance with embodiments of the invention may be made as follows: On one surface of a base (e.g., a non-woven fabric), a pressure-sensitive adhesive composition is applied to make a pressure-sensitive adhesive layer. Then, the surface of the adhesive layer is covered with a cover. Die cutting is then performed to produce individual pressure-sensitive adhesive structures. Preferably, cutting of the cover to give a small piece and a large piece (or two or more equal or unequal pieces) is also performed. Note that while a kit in accordance with embodiments of the invention contain two or more pressure-sensitive adhesive structures, these two or more pieces may be pre-cut (scored) but are stilled attached to each other - i.e., they may come as a "single" piece that are easily separated into individual pieces by a user. Therefore, in this description, "two or more" pieces are intended to include such "attached" pieces.

### Effect of Invention

According to embodiments of the present invention, a kit that includes an exothermic structure having at least two pressure-sensitive adhesive parts on its ends (edges) can be readily prepared at low costs. There is no need to use an expensive machine.

During the use of an exothermic structure that is made from a kit of the present invention, risks of low-temperature burn are low. This is because in accordance with embodiments of the invention an adhesive is not applied all over the surface of the exothermic structure, and therefore the exothermic structure would not adhere to skin tightly. Instead, an exothermic structure of the invention is adhere to the skin only by two or more ends where there are no exothermic composition.

When an exothermic structure is peeled off after use, it gives less tingle or discomfort. This is because the adhesive is applied over a relatively small area.

According to methods of the present invention, users can readily adhere an exothermic structure to a site of application, such as skin or outside of underwear.

Hereafter, the present invention will be explained with reference to the following examples.

### Example 1: Preparation of pressure-sensitive adhesive structures

On a spun lace non-woven fabric (manufactured by Asahi Kasei Corporation; basis weight: 40 g/m²) made of a polyester and rayon (80:20 by weight) and having a beige color, a pressure-sensitive adhesive of a styrene-isoprene-styrene block copolymer type (ME126; manufactured by Nippon NSC Ltd.) was applied to make a pressure-sensitive adhesive layer having a thickness of about 30µm. The surface of the adhesive layer was covered with a polyester film having a silicone coating layer (Cerapeel; manufactured by Toray Advanced Film co., Ltd.; thickness: 38µm).

From the obtained structure, individual adhesive structures having the form shown in Figure 6 were cut by using a die-cut apparatus having a Thomson-type blade. At the same time, the cover was cut to give a small piece *s* and a large piece *l*. Thus, a pressure-sensitive adhesive structure having a length of 9.5 cm and a width (the length of the widest part) of 2.5 cm was obtained in this example.

### Example 2: Preparation of exothermic structures and kits

To a spun lace non-woven fabric (manufactured by Asahi Kasei Corporation; basis weight: 40 g/m²) made of a polyester and rayon (80:20 by weight) and having a white color, a commercially-available, air-impermeable polyethylene film (manufactured by Fukui Minasell Co., Ltd.; thickness: 40µm) was adhered by heat-sealing. Thus, a sheet-like structure A was obtained.

To a spun lace non-woven fabric (manufactured by Asahi Kasei Corporation; basis weight: 60 g/m²) made of a polyester and having a beige color, a porous polyethylene film (TSF-EU; manufactured by Kohjin Co., Ltd; thickness: 50µm) was partly adhered. Thus, a sheet-like structure B was obtained. The moisture-permeability [JIS Z 0208 (1976)] of the sheet-like structure B, as determined by the Lyssy method, was about 310 g/m². 24 hours.

The sheet-like structure A was then heat-sealed to the sheet-like structure B in a lateral direction, wherein the air-impermeable polyethylene film faced the porous polyethylene film. Then, the structures A and B were heat-sealed to each other in the longitudinal direction at both sides so as to make a pouch with three sides sealed. Then, 20 g of the exothermic composition having a formula shown in Table 1 was put into the pouch through the open side.

Afterwards, the structures A and B were laterally heat-sealed to each other to close the open side and form a sealed pouch that contains the exothermic composition. The place where the exothermic composition existed was pressed into a flat configuration having a thickness of about 1.5 mm. Thereafter, the same operations were repeated for each exothermic structure. Alternatively, several of the exothermic structures (e.g., side-by-side in a row) may be prepared simultaneously, especially with a machine.

When the preparation of individual structures is finished, these structures may be cut into individual units. At the center of the longitudinal direction of the laterally heat-sealed portion, cutting may be performed to separate the individual exothermic structures. The exothermic structure, for example, may have a size of 13 cm×9.5 cm with the width of the peripheral heat-sealed portions, i.e., ends (or edges), of 6 mm.

This exothermic structure and two pressure-sensitive adhesive structures that had been made in Example 1 were promptly put into an air-impermeable bag that was made from a commercially-available material (e.g., a laminate film of a cast polypropylene (CPP) and a polyethylene). Then, the bag was promptly heat-sealed to form a kit of the invention.

**Table 1**

| Names of Raw Materials | Amounts (wt. %) |
|---|---|
| Iron Powder | 60 |
| Active Carbon | 5 |
| Carboxymethyl Cellulose | 2 |
| Acrylic Acid Starch | 2 |
| Sodium Chloride | 2 |
| Water | 29 |
| Total | 100 |

### Example 3: Use of the kits

In a test, three men and three women (test subjects) (age: 20 through 50 years old) used the kits prepared in Example 2.

The test subjects opened the air-impermeable bag to remove the exothermic structure and two pressure-sensitive adhesive structures. They peeled off the small piece of the cover of one adhesive structure and then applied the exposed pressure-sensitive adhesive layer onto one end along one short side (end *b* in Figure 1) of the air-permeable side of the exothermic structure. Similarly, other pressure-sensitive adhesive structure was applied onto the other end along the other short side (end d in Figure 1). All test subjects applied the pressure-sensitive adhesive structures onto the air-permeable side of the exothermic structure without stumbling or difficulty.

The test subjects applied the exothermic structure having two pressure-sensitive adhesive structures onto hypogastrium, while peeling off the large piece of the covers to expose the pressure-sensitive adhesive layers.

After 8 hours, they peeled off the exothermic structures. No test subject had any low-temperature burn. When the exothermic structure was peeled off, few body hairs were pulled. Only a man reported a feeling of a tingle sensation.

Advantages of embodiments of the invention may include one or more of the following. Kits in accordance with embodiments of the invention may be manufactured with relative ease and at low costs because they do not require expensive equipment. A kit of the invention may be used by a user with relatively ease. In addition, a kit of the invention may be used with less discomfort or risk of low-temperature bums because it uses less adhesive to attach the exothermic structure to the skin and would not attach the structure too tightly to the skin.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

## Claims

1. A kit comprising an exothermic structure, at least two pressure-sensitive adhesive structures, and an air-impermeable bag, wherein the exothermic structure comprises a pouch having an air-permeable side and an air-impermeable side, and an exothermic composition that is sealed in the pouch, the pressure-sensitive adhesive structures each comprises a base, a pressure-sensitive adhesive layer, and a cover in this order, and the exothermic structure is by itself, or together with the adhesive structures, sealed in the air-impermeable bag.

2. The kit according to claim 1, wherein the cover is cut to give a small piece and a large piece.

3. The kit according to claim 1, wherein the cover is cut into two or more pieces.

4. The kit according to claim 1, wherein the air-permeable side and the air-impermeable side can be visually distinguished from each other.

5. The kit according to claim 4, wherein an outer surface of the air-permeable side and an outer surface of the air-impermeable side have different colors or patterns.

6. The kit according to claim 5, wherein a surface of the base, opposite the surface having the pressure-sensitive adhesive layer, has a color or pattern that is identical to the color or pattern on the outer surface of the air-permeable side of the exothermic structure.

7. The kit according to claim 1, wherein the kit comprises two, three, or four pressure-sensitive adhesive structures.

8. A method for using the kit of claim 1, comprising:
taking the exothermic structure out from the air-impermeable bag,
peeling off parts of the covers of the pressure-sensitive adhesive structures to expose parts of surfaces of the pressure-sensitive adhesive layers,
pressing the bases of the pressure-sensitive adhesive structures to adhere the exposed pressure-sensitive adhesive layers to ends of the air-permeable side of the exothermic structure,
peeling off the remaining covers to expose the remaining surfaces of the pressure-sensitive adhesive layers, and
pressing the bases of the pressure-sensitive adhesive structures to adhere the exposed pressure-sensitive adhesive layers to a site of application.

9. The method according to claim 8, wherein each cover is cut to give a small piece and a large piece, and the small piece is pealed off first.

10. The method of claim 8, wherein the site of application is skin or outside of underwear.
